(19)

Europäisches
Patentamt
European
Patent Office
Office européen
des brevets

(11) **EP 4 423 863 B1**

(12) **EUROPEAN PATENT SPECIFICATION**

(45) Date of publication and mention
of the grant of the patent:
**15.04.2026 Bulletin 2026/16**

(21) Application number: **22812932.6**

(22) Date of filing: **25.10.2022**

(51) International Patent Classification (IPC):
***H01S 3/00*** ^(2006.01)

(52) Cooperative Patent Classification (CPC):
**H01S 3/2308; A61B 18/245; A61B 2018/263;**
**A61B 2018/266; H01S 3/10015**

(86) International application number:
**PCT/US2022/047691**

(87) International publication number:
**WO 2023/076241 (04.05.2023 Gazette 2023/18)**

(54) **HIGH BANDWIDTH ENERGY SOURCE FOR IMPROVED TRANSMISSION THROUGH OPTICAL FIBER FOR INTRAVASCULAR LITHOTRIPSY**

HOCHBANDBREITENENERGIEQUELLE FÜR VERBESSERTE ÜBERTRAGUNG DURCH GLASFASER FÜR INTRAVASKULÄRE LITHOTRIPSIE

SOURCE D'ÉNERGIE À LARGEUR DE BANDE ÉLEVÉE POUR UNE TRANSMISSION AMÉLIORÉE À TRAVERS UNE FIBRE OPTIQUE POUR LA LITHOTRITIE ENDOVÉSICALE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **28.10.2021 US 202163273065 P**
**20.10.2022 US 202217970359**

(43) Date of publication of application:
**04.09.2024 Bulletin 2024/36**

(73) Proprietor: **Bolt Medical, Inc.**
**Carlsbad, CA 92008 (US)**

(72) Inventors:
• **COOK, Christopher, A.**
**Laguna Niguel, CA 92677 (US)**

• **BACHER, Gerald, David**
**Carlsbad, CA 92008 (US)**
• **CUMMINS, Caroline**
**Bozeman, MT 59718 (US)**
• **BESNIER, Alexandre**
**22300 Lannion (FR)**
• **MAINE, Patrick**
**22300 Lannion (FR)**

(74) Representative: **Vossius & Partner**
**Patentanwälte Rechtsanwälte mbB**
**P.O. Box 86 07 67**
**81634 München (DE)**

(56) References cited:
**US-A1- 2008 086 118      US-A1- 2014 188 094**
**US-A1- 2015 250 542      US-A1- 2021 212 765**
**US-A1- 2021 298 603**

## Description

## BACKGROUND

Vascular lesions within vessels in the body can be associated with an increased risk for major adverse events, such as myocardial infarction, embolism, deep vein thrombosis, stroke, and the like. Severe vascular lesions can be challenging to treat and achieve patency for a physician in a clinical setting. Vascular lesions may be treated using interventions such as drug therapy, balloon angioplasty, atherectomy, stent placement, vascular graft bypass, to name a few. Such interventions may not always be ideal or may require subsequent treatment to address the lesion.

Using optical fiber delivery of laser pulses to generate plasma-induced mechanical impulses on the lesions is one way to treat vascular lesions by fragmentation. Shaping the temporal form of the optical pulse makes it possible to reduce the peak power of the pulse below the damage threshold of the optical fiber and still transmit full energy for generating the mechanical impulse. This increases the amount of energy that can be delivered in a set time interval while minimizing the peak laser intensity to remain below the damage threshold of the optical fiber. However, nonlinear optical processes in the bulk optical fiber may still throttle back energy transmission through the fiber. Thus, nonlinear optical processes can limit the peak energy delivered and the device's ability to fracture lesions. In this context, documents US 2015/250542 and US 2014/188094 A1 are referred to.

One of the principal issues encountered with using optical energy pulses to generate plasma-driven acoustic bubbles is coupling sufficient energy into an optical fiber's input end (proximal end) or other light guide to create an effective therapeutic effect at an output end (distal end) of the optical fiber. Damage to the light guide that transmits the optical energy pulse inside the body has been a critical challenge in developing this technology. The factors involved are the Laser-Induced Damage Threshold (LIDT) of the light guide interfaces and the bulk threshold of the medium itself. The LIDT for bulk fused silica is 1866 J cm$^{-2}$ at 1064 nm for a 12 ns pulse. The LIDT for surfaces can be one-tenth of this or less. Surface finish quality and cleanliness significantly impact this number. Since the damage threshold for the light guide surface is much lower than that for bulk material, failures typically appear as damage on the light guide's input (proximal) end. The amount of energy transmitted through the light guide is then limited by the peak intensity of the pulse on the proximal surface. This limitation has been addressed primarily by stretching the energy pulse width temporally. This reduces peak power and surface irradiance while maintaining overall energy.

Additionally, recent experiments have indicated that for pulses with an approximately Gaussian temporal shape, the peak pressure generated by the optical pulse is proportional to the peak intensity of the pulse. This means that even though shorter pulses are limited to lower total energies by the damage threshold of the transmission medium, they can produce similar pressure waves to longer pulses using significantly less total optical energy. The result is that sufficiently energetic pressure waves can be created to fracture calcified lesions while remaining well below the damage threshold of the light guide.

Even using the technique of pulse stretching to reduce peak power at the light guide surface, the peak power remains sufficiently high to induce Stimulated Brillouin Scattering (SBS) in the light guide material itself. SBS is a nonlinear process that can occur in an optical medium at relatively low input power levels. It manifests itself by generating a backward-propagating Stokes wave that carries most of the input power once the Brillouin threshold is reached. The interaction of the input photon and the moving refractive index variations within the material creates a backward scattered phonon, the Stokes wave. This process removes energy from the input beam. In turn, the backward propagating wave creates a region of periodic index variation that behaves like a Bragg grating. This scatters more of the forward beam backward. This nonlinear process creates an exponential decrease in total transmitted energy with incremental increase input. It fundamentally throttles back the total energy transmitted as more is put in.

For the case where the input light frequency linewidth, $\Delta u_p$, is narrow compared to the frequency linewidth of Brillouin scatter, the unsaturated Brillouin gain coefficient, $G_{B0}$, at wavelength $\lambda$ is given by:

$$G_{B0} = \frac{2\pi n^7 p_{12}^2}{c\lambda^2 \rho V_s \Delta v_B}$$

where $p_{12}$ is the elasto-optical coefficient of the material, $\rho$ is its density, $n$ is its refractive index, $V_s$ is the acoustic velocity of the vibrations, and $\Delta u_B$ is the Brillouin frequency linewidth in the material. The Brillouin frequency linewidth in silica is around 135 MHz. At $\lambda = 1\mu m$, $G_{B0} = 4.5 \times 10^{-9} cm/W$. If the input photon beam linewidth, $\Delta u_p$, is larger than

$$G_B = G_{B0} \frac{\Delta v_B}{\Delta v_p}$$

This indicates directly that the one way to reduce the effects of SBS is to significantly broaden $\Delta u_p$.

Linewidth (optical bandwidth) is a measure of spectral purity (monochromaticity). In general, pulsed solid-state lasers with short cavity lengths have characteristically narrow linewidths, on the order of tens of kHz to hundreds of MHz. It is also helpful to consider this in terms of coherence length given by:

$$L_c = \frac{c}{\pi \Delta v_p}$$

This range of linewidths corresponds to a coherence length from around thousands of meters down to 2 m. This is the range over which laser light can interfere with itself and contribute to SBS. The 135 MHz Brillouin frequency linewidth in silica corresponds to a coherence length around 70 cm. To reduce the Brillouin gain coefficient to the point where SBS would not impact light transmission through the fiber, the linewidth needs to be several orders of magnitude greater than the basic Brillouin frequency linewidth. That would be 13 GHz to 30 GHz, corresponding to a coherence length around 7 mm to 3 mm. Other energy sources can have bandwidths ranging from 50 pm to 69 pm, corresponding to 13.25 GHz to 18.25 GHz at 1064 nm.

## SUMMARY

The present invention is directed toward a catheter system for treating a treatment site within or adjacent to a vessel wall or heart valve. In various embodiments, the catheter system includes a light guide and a light source. The light guide is configured to selectively receive light energy. The light source generates the light energy. The light source is in optical communication with the light guide. The light source can include (i) a seed source that outputs the light energy, (ii) a pre-amplifier that receives the light energy from the seed source, the pre-amplifier being in optical communication with the seed source, and (iii) an amplifier that receives the light energy from the pre-amplifier, the amplifier being in optical communication with the pre-amplifier and the light guide.

In some embodiments, the catheter system further includes a seed controller that controls the seed source.

In certain embodiments, the catheter system further includes an optical element that is configured to direct the light energy into the light guide.

In various embodiments, the seed source includes one of a diode laser, a programmable semiconductor laser, a gated fiber optic laser, and a low power solid-state laser.

In some embodiments, the seed source, the pre-amplifier, and the amplifier are free space coupled within the light source.

In certain embodiments, the seed source is optically coupled to the pre-amplifier with a first coupling light guide, and the pre-amplifier is optically coupled to the amplifier with a second coupling light guide.

In various embodiments, the pre-amplifier includes one of a fiber optic laser, a solid-state laser, a flashlamp, and a diode pumped neodymium-doped yttrium aluminum garnet rod.

In some embodiments, the amplifier includes one of a high gain stage that is configured to have a high energy output capability, a fiber optic laser, a diode pumped solid-state laser, and a flashlamp.

In certain embodiments, the amplifier includes a gain medium including one of (i) a neodymium-doped yttrium aluminum garnet rod, (ii) a neodymium-doped yttrium aluminum garnet slab, (iii) a neodymium-doped glass, and (iv) an erbium-doped yttrium lithium fluoride, the gain medium being optically coupled to one of a laser diode stack and a flashlamp.

The light source includes a collimator that collimates the light energy output by the pre-amplifier, the collimator being in optical communication with the pre-amplifier and the amplifier.

The present invention is also directed toward a catheter system for treating a treatment site within or adjacent to a vessel wall or heart valve. In various embodiments, the catheter system includes a light guide and a light source. The light guide is configured to selectively receive light energy. The light source generates the light energy. The light source is in optical communication with the light guide. The light source can include (i) a seed source that outputs the light energy, (ii) a linewidth modifier that modifies a linewidth of the light energy output by the seed source, (iii) a pre-amplifier that receives the light energy from the linewidth modifier, the pre-amplifier being in optical communication with the linewidth modifier, (iv) a collimator that collimates the light energy output by the pre-amplifier, the collimator being in optical communication with the pre-amplifier, and (v) an amplifier that receives the light energy from the pre-amplifier, the amplifier being in optical communication with the collimator and the light guide.

In some embodiments, the seed source includes one modulated distributed feedback laser.

In certain embodiments, the seed source includes a plurality of modulated distributed feedback lasers.

In various embodiments, the plurality of modulated distributed feedback lasers are configured to have a seed offset in center wavelengths that is above and below an amplifier wavelength of the amplifier.

In some embodiments, the seed source is optically coupled to the linewidth modifier with a first coupling light guide.

In certain embodiments, a seed pulse shape of the seed source is at least partially controlled by directly modulating the seed source.

In various embodiments, a seed pulse shape of the seed source is at least partially controlled by an acousto-optic modulator.

In some embodiments, the seed source includes a diode that is configured to have a high spatial coherence and a low temporal coherence.

In certain embodiments, the diode is a superluminescent diode.

In various embodiments, the linewidth modifier is a band-limiting filter.

In some embodiments, the linewidth modifier is a fiber-optic Bragg grating.

In certain embodiments, the seed source and the linewidth modifier work in conjunction to (i) increase a seed linewidth of the seed source, (ii) improve amplification of the light energy, and (iii) minimize Stimulated Brillouin Scattering in the light guide.

The present invention is further directed toward a catheter system for treating a treatment site within or adjacent to a vessel wall or heart valve. In various embodiments, the catheter system includes a light guide and a light source. The light guide is configured to selectively receive light energy. The light source generates the light energy. The light source is in optical communication with the light guide. The light source can include (i) a seed source that outputs light energy, and (ii) an amplifier that receives the light energy from the seed source, the amplifier being in optical communication with the seed source and the light guide.

The present invention is also directed toward a method for treating a treatment site within or adjacent to a vessel wall or a heart valve that includes providing and/or using any of the catheter systems shown and/or described herein.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims.

## BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:

>   Figure 1 is a simplified schematic diagram of one embodiment of a portion of a catheter system having features of the present invention;
>   Figure 2 is a simplified schematic diagram of another embodiment of a portion of the catheter system;
>   Figure 3 is a simplified schematic diagram of yet another embodiment of a portion of the catheter system; and
>   Figure 4 is a simplified schematic diagram of yet another embodiment of a portion of the catheter system.

While embodiments are susceptible to various modifications and alternative forms, specifics thereof have been shown by way of example and drawings, and will be described in detail. However, it should be understood that the scope herein is not limited to the particular aspects described.

## DESCRIPTION

Treatment of vascular lesions (also referred to herein as "treatment sites") can reduce major adverse events or death in affected subjects. As referred to herein, a major adverse event is one that can occur anywhere within the body due to the presence of a vascular lesion. Major adverse events can include but are not limited to, major adverse cardiac events, major adverse events in the peripheral or central vasculature, major adverse events in the brain, major adverse events in the musculature, or major adverse events in any of the internal organs.

As used herein, the terms "intravascular lesion," "vascular lesion," and "treatment site" are used interchangeably unless otherwise noted. The intravascular lesions and/or the vascular lesions are sometimes referred to herein simply as "lesions." Also, as used herein, the terms "focused location" and "focused spot" can be used interchangeably unless otherwise noted and can refer to any location where the light energy is focused to a small diameter than the initial diameter of the light source.

Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention, as illustrated in the accompanying drawings.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is appreciated that such a development effort might be complex and time-consuming, but would nevertheless be a routine undertaking of engineering for those of ordinary skill in the art having the benefit of this disclosure.

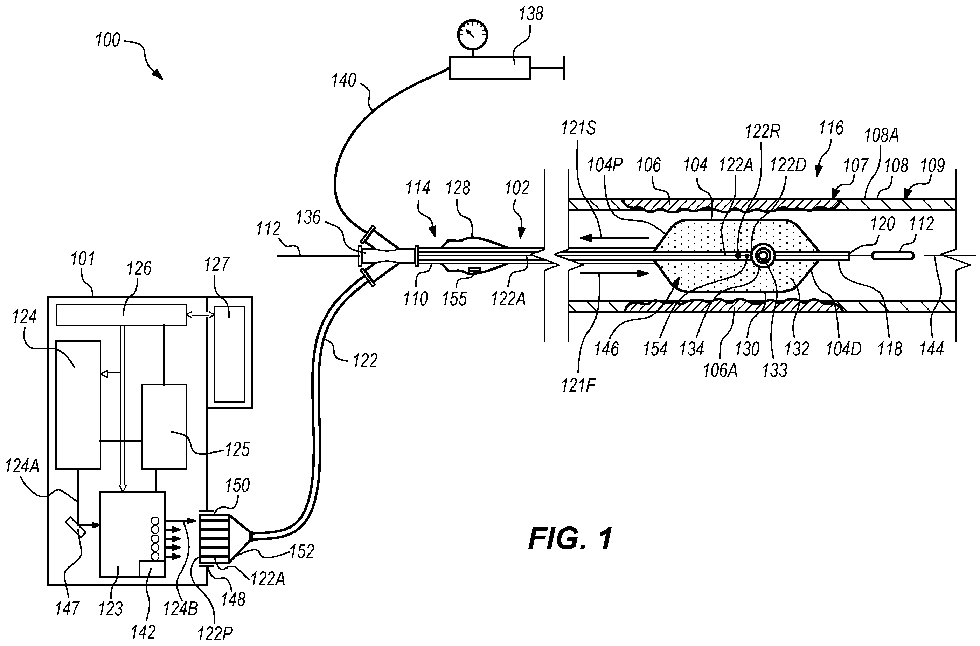

The catheter systems disclosed herein can include many different forms. Referring now to Figure 1, a schematic cross-sectional view is shown of a catheter system 100 in accordance with various embodiments. The catheter system 100 is suitable for imparting pressure waves to induce fractures in one or more treatment sites within or adjacent to a vessel wall of a blood vessel or on or adjacent to a heart valve within a body of a patient. In the embodiment illustrated in Figure 1, the catheter system 100 can include one or more of a catheter 102, a light guide bundle 122 including one or more light guides 122A (some embodiments described here include at least a first light guide and a second light guide), a handle assembly 128, a source manifold 136, a fluid pump 138 and a system console 101. The system console 101 can include one or more of a multiplexer 123, a light source 124, a power source 125, a system controller 126, a graphic user interface 127 (also sometimes referred to herein as a "GUI"), and an optical analyzer assembly 142. Alternatively, the catheter system 100 can include greater or fewer components than those specifically illustrated and described in relation to Figure 1.

It is appreciated that while the catheter system 100 is generally described herein as including a light guide bundle 122, including one or more light guides 122A, and a light source 124. In some alternative embodiments, the catheter system 100 can include an energy guide bundle that includes different types of energy guides and/or a different type of energy source.

In various embodiments, the catheter 102 is configured to move to a treatment site 106 within or adjacent to a vessel wall 108A of a blood vessel 108 within a body 107 of a patient 109. The treatment site 106 can include one or more vascular lesions 106A, such as calcified vascular lesions, for example. Additionally, or in the alternative, the treatment site 106 can include vascular lesions 106A such as fibrous vascular lesions. Still alternatively, in some implementations, the catheter 102 can be used at a treatment site 106 within or adjacent to a heart valve within the body 107 of the patient 109.

The catheter 102 can include an inflatable balloon 104 (sometimes referred to herein simply as a "balloon"), a catheter shaft 110, and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter shaft 110 can also include a guidewire lumen 118, which is configured to move over the guidewire 112. As utilized herein, the guidewire lumen 118 defines a conduit through which the guide wire 112 extends. The catheter shaft 110 can further include an inflation lumen (not shown) and/or various other lumens for various other purposes. In some embodiments, the catheter 102 can have a distal end opening 120 and can accommodate and be tracked over the guidewire 112 as the catheter 102 is moved and positioned at or near the treatment site 106. In some embodiments, the balloon proximal end 104P can be coupled to the catheter shaft 110, and the balloon distal end 104D can be coupled to the guidewire lumen 118.

The balloon 104 includes a balloon wall 130 that defines a balloon interior 146. The balloon 104 can be selectively inflated with a balloon fluid 132 to expand from a deflated state suitable for advancing the catheter 102 through a patient's vasculature to an inflated state (as shown in Figure 1) suitable for anchoring the catheter 102 in position relative to the treatment site 106. Stated in another manner, when the balloon 104 is in the inflated state, the balloon wall 130 of the balloon 104 is configured to be positioned substantially adjacent to the treatment sites 106. It is appreciated that although Figure 1 illustrates the balloon wall 130 of the balloon 104 is shown spaced apart from the treatment site 106 of the blood vessel 108 when in the inflated state, this is done merely for ease of illustration. It is recognized that the balloon wall 130 of the balloon 104 will typically be substantially directly adjacent to and/or abutting the treatment site 106 when the balloon 104 is in the inflated state.

The balloon 104 suitable for use in the catheter system 100 includes those that can be passed through the vasculature of a patient 109 when in the deflated state. In some embodiments, the balloon 104 is made from silicone. In other embodiments, the balloon 104 can be made from polydimethylsiloxane (PDMS), polyurethane, polymers such as PEBAX™ material, nylon, or any other suitable material.

The balloon 104 can have any suitable diameter (in the inflated state). In various embodiments, the balloon 104 can have a diameter (in the inflated state) ranging from less than one millimeter (mm) up to 25 mm. In some embodiments, the balloon 104 can have a diameter (in the inflated state) ranging from at least 1.5 mm up to 14 mm. In some embodiments, the balloons 104 can have a diameter (in the inflated state) ranging from at least two mm up to five mm.

In some embodiments, the balloon 104 can have a length ranging from at least three mm to 300 mm. More particularly, in some embodiments, the balloon 104 can have a length ranging from at least eight mm to 200 mm. It is appreciated that a balloon 104 having a relatively longer length can be positioned adjacent to larger treatment sites 106 and, thus, may be used for imparting pressure waves onto and inducing fractures in larger vascular lesions 106A or multiple vascular lesions 106A at precise locations within the treatment site 106. It is further appreciated that a longer balloon 104 can also be positioned adjacent to multiple treatment sites 106 at any one given time.

The balloon 104 can be inflated to inflation pressures of between approximately one atmosphere (atm) and 70 atm. In some embodiments, the balloon 104 can be inflated to inflation pressures of from at least 20 atm to 60 atm. In other embodiments, the balloon 104 can be inflated to inflation pressures of from at least six atm to 20 atm. In still other embodiments, the balloon 104 can be inflated to inflation pressures of from at least three atm to 20 atm. In yet other embodiments, the balloon 104 can be inflated to inflation pressures of from at least two atm to ten atm.

The balloon 104 can have various shapes, including, but not to be limited to, a conical shape, a square shape, a rectangular shape, a spherical shape, a conical/square shape, a conical/spherical shape, an extended spherical shape, an oval shape, a tapered shape, a bone shape, a stepped diameter shape, an offset shape, or a conical offset shape. In some embodiments, the balloon 104 can include a drug-eluting coating or a drug-eluting stent structure. The drug-eluting coating or drug-eluting stent can include one or more therapeutic agents, including anti-inflammatory agents, anti-neoplastic agents, anti-angiogenic agents, and the like.

The balloon fluid 132 can be a liquid or a gas. Some examples of the balloon fluid 132 suitable for use can include, but are not limited to, one or more of water, saline, contrast medium, fluorocarbons, perfluorocarbons, gases, such as carbon dioxide, or any other suitable balloon fluid 132. In some embodiments, the balloon fluid 132 can be used as a base inflation fluid. In some embodiments, the balloon fluid 132 can include a mixture of saline to contrast medium in a volume ratio of approximately 50:50. In other embodiments, the balloon fluid 132 can include a mixture of saline to contrast medium in a volume ratio of approximately 25:75. In still other embodiments, the balloon fluid 132 can include a mixture of saline to contrast medium in a volume ratio of approximately 75:25. However, it is understood that any suitable ratio of saline to contrast medium can be used. The balloon fluid 132 can be tailored based on composition, viscosity, and the like so that the rate of travel of the pressure waves are appropriately manipulated. In certain embodiments, the balloon fluid 132 suitable for use herein is biocompatible. A volume of balloon fluid 132 can be tailored by the chosen light source 124 and the type of balloon fluid 132 used.

In some embodiments, the contrast agents used in the contrast media can include but are not limited to iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine-based contrast agents can be used. Suitable non-iodine-containing contrast agents can include gadolinium (III)-based contrast agents. Suitable fluorocarbon and perfluorocarbon agents can include but are not limited to agents such as perfluorocarbon dodecafluoropentane (DDFP, C5F12).

The balloon fluids 132 can include those that include absorptive agents that can selectively absorb light in the ultraviolet region (e.g., at least ten nanometers (nm) to 400 nm), the visible region (e.g., at least 400 nm to 780 nm), or the near-infrared region (e.g., at least 780 nm to 2.5 $\mu$m) of the electromagnetic spectrum. Suitable absorptive agents can include those with absorption maxima along the spectrum from at least ten nm to 2.5 $\mu$m. Alternatively, the balloon fluid 132 can include absorptive agents that can selectively absorb light in the mid-infrared region (e.g., at least 2.5 $\mu$m to 15 $\mu$m) or the far-infrared region (e.g., at least 15 $\mu$m to one mm) of the electromagnetic spectrum. In various embodiments, the absorptive agent can be those that have an absorption maximum matched with the emission maximum of the laser used in the catheter system 100. By way of non-limiting examples, various lasers described herein can include neodymium:yttrium-aluminum-garnet (Nd:YAG - emission maximum = 1064 nm) lasers, holmium:YAG (Ho:YAG - emission maximum = 2.1 $\mu$m) lasers, or erbium:YAG (Er:YAG - emission maximum = 2.94 $\mu$m) lasers. In some embodiments, the absorptive agents can be water-soluble. In other embodiments, the absorptive agents are not water-soluble. In some embodiments, the absorptive agents used in the balloon fluids 132 can be tailored to match the peak emission of the light source 124. Various light sources 124 having emission wavelengths of at least ten nanometers to one millimeter are discussed elsewhere herein.

The catheter shaft 110 of the catheter 102 can be coupled to the one or more light guides 122A of the light guide bundle 122 that are in optical communication with the light source 124. The light guide(s) 122A can be disposed along the catheter shaft 110 and within the balloon 104. Each of the light guides 122A can have a guide distal end 122D that is at any suitable longitudinal position relative to a length of the balloon 104. In some embodiments, each light guide 122A can be an optical fiber, and the light source 124 can be a laser. The light source 124 can be in optical communication with the light guides 122A at the proximal portion 114 of the catheter system 100. More particularly, the light source 124 can selectively, simultaneously, sequentially, and/or be in optical communication with each of the light guides 122A in any desired combination, order, and/or pattern due to the presence and operation of the handle assembly 128.

In some embodiments, the catheter shaft 110 can be coupled to multiple light guides 122A such as a first light guide, a second light guide, a third light guide, etc., which can be disposed at any suitable positions about the guidewire lumen 118 and/or the catheter shaft 110. For example, in certain non-exclusive embodiments, two light guides 122A can be spaced apart by approximately 180 degrees about the circumference of the guidewire lumen 118 and/or the catheter shaft 110; three light guides 122A can be spaced apart by approximately 120 degrees about the circumference of the guidewire lumen 118 and/or the catheter shaft 110, or four light guides 122A can be spaced apart by approximately 90 degrees about the circumference of the guidewire lumen 118 and/or the catheter shaft 110. Still alternatively, multiple light guides 122A need not be uniformly spaced apart from one another about the circumference of the guidewire lumen 118 and/or the catheter shaft 110. More particularly, the light guides 122A can be disposed either uniformly or non-uniformly about the guidewire lumen 118 and/or the catheter shaft 110 to achieve the desired effect in the desired locations.

The catheter system 100 and/or the light guide bundle 122 can include any number of light guides 122A in optical communication with the light source 124 at the proximal portion 114, and with the balloon fluid 132 within the balloon interior 146 of the balloon 104 at the distal portion 116. For example, in some embodiments, the catheter system 100 and/or the light guide bundle 122 can include from one light guide 122A to five light guides 122A. In other embodiments, the catheter system 100 and/or the light guide bundle 122 can include from five light guides 122A to fifteen light guides 122A. In yet other embodiments, the catheter system 100 and/or the light guide bundle 122 can include from ten light guides 122A to thirty light guides 122A. Alternatively, in still other embodiments, the catheter system 100 and/or the light guide bundle 122 can include greater than 30 light guides 122A.

The light guides 122A can have any suitable design for purposes of generating plasma and/or pressure waves in the balloon fluid 132 within the balloon interior 146. In certain embodiments, the light guides 122A can include an optical fiber or flexible light pipe. The light guides 122A can be thin and flexible and can allow light signals to be sent with very little loss of strength. The light guides 122A can include a core surrounded by a cladding about its circumference. In some embodiments, the core can be a cylindrical core or a partially cylindrical core. The core and cladding of the light guides 122A can be formed from one or more materials, including but not limited to one or more types of glass, silica, or one or more polymers. The light guides 122A may also include a protective coating, such as a polymer. It is appreciated that the index of refraction of the core will be greater than the index of refraction of the cladding.

Each light guide 122A can guide light energy along its length from a guide proximal end 122P to the guide distal end 122D, having at least one optical window (not shown) that is positioned within the balloon interior 146.

In various embodiments, the guide distal end 122D can further include and/or incorporate a distal light receiver 122R that enables light energy to be moved back into and through the light guide 122A from the guide distal end 122D to the guide proximal end 122P. Stated another way, the light energy can move in a first direction 121F along the light guide 122A that is generally from the guide proximal end 122P toward the guide distal end 122D of the light guide 122A. At least a portion of the light energy can also move in a second direction 121S along the light guide 122A that is substantially opposite the first direction 121F, i.e., from the guide distal end 122D toward the guide proximal end 122P of the light guide 122A. Moreover, as described in greater detail hereinbelow, the light energy emitted from the guide proximal end 122P after being moved back through the light guide 122A (in the second direction 121S) can be separated and then optically detected, interrogated, and/or analyzed through use of the optical analyzer assembly 142.

The light guides 122A can assume many configurations about and/or relative to the catheter shaft 110 of the catheter 102. In some embodiments, the light guides 122A can run parallel to the longitudinal axis 144 of the catheter shaft 110. In some embodiments, the light guides 122A can be physically coupled to the catheter shaft 110. In other embodiments, the light guides 122A can be disposed along a length of an outer diameter of the catheter shaft 110. In yet other embodiments, the light guides 122A can be disposed within one or more light guide lumens within the catheter shaft 110.

The light guides 122A can also be disposed at any suitable positions about the circumference of the guidewire lumen 118 and/or the catheter shaft 110, and the guide distal end 122D of each of the light guides 122A can be disposed at any suitable longitudinal position relative to the length of the balloon 104 and/or relative to the length of the guidewire lumen 118 to more effectively and precisely impart pressure waves for purposes of disrupting the vascular lesions 106A at the treatment site 106.

In certain embodiments, the light guides 122A can include one or more photoacoustic transducers 154, where each photoacoustic transducer 154 can be in optical communication with the light guide 122A within which it is disposed. In some embodiments, the photoacoustic transducers 154 can be in optical communication with the guide distal end 122D of the light guide 122A. Additionally, in such embodiments, the photoacoustic transducers 154 can have a shape that corresponds with and/or conforms to the guide distal end 122D of the light guide 122A.

The photoacoustic transducer 154 is configured to convert light energy into an acoustic wave at or near the guide distal end 122D of the light guide 122A. The direction of the acoustic wave can be tailored by changing an angle of the guide distal end 122D of the light guide 122A.

In certain embodiments, the photoacoustic transducers 154 disposed at the guide distal end 122D of the light guide 122A can assume the same shape as the guide distal end 122D of the light guide 122A. For example, in certain non-exclusive embodiments, the photoacoustic transducer 154 and/or the guide distal end 122D can have a conical shape, a convex shape, a concave shape, a bulbous shape, a square shape, a stepped shape, a half-circle shape, an ovoid shape, and the like. The light guide 122A can further include additional photoacoustic transducers 154 disposed along one or more side surfaces of the length of the light guide 122A.

In some embodiments, the light guides 122A can further include one or more diverting features or "diverters" (not shown in Figure 1) within the light guide 122A that are configured to direct light to exit the light guide 122A toward a side surface which can be located at or near the guide distal end 122D of the light guide 122A, and toward the balloon wall 130. A diverting feature can include any system feature that diverts light energy from the light guide 122A away from its axial path toward a side surface of the light guide 122A. Additionally, the light guides 122A can each include one or more light windows disposed along the longitudinal or circumferential surfaces of each light guide 122A and in optical communication with a diverting feature. Stated in another manner, the diverting features can be configured to direct light energy in the light guide 122A toward a side surface that is at or near the guide distal end 122D, where the side surface is in optical communication with a light window. The light windows can include a portion of the light guide 122A that allows light energy to exit the light guide 122A from within the light guide 122A, such as a portion of the light guide 122A lacking a cladding material on or about the light guide 122A.

Examples of the diverting features suitable for use include a reflecting element, a refracting element, and a fiber diffuser. The diverting features suitable for focusing light energy away from the tip of the light guides 122A can include but are not to be limited to, those having a convex surface, a gradient-index (GRIN) lens, and a mirror focus lens. Upon contact with the diverting feature, the light energy is diverted within the light guide 122A to one or more of a plasma generator 133 and the photoacoustic transducer 154 that is in optical communication with a side surface of the light guide 122A. As noted, the photoacoustic transducer 154 then converts light energy into an acoustic wave that extends away from the side surface of the light guide 122A.

The source manifold 136 can be positioned at or near the proximal portion 114 of the catheter system 100. The source manifold 136 can include one or more proximal end openings that can receive the one or more light guides 122A of the light guide bundle 122, the guidewire 112, and/or an inflation conduit 140 that is coupled in fluid communication with the fluid pump 138. The catheter system 100 can also include the fluid pump 138 that is configured to inflate the balloon 104 with the balloon fluid 132 as needed.

As noted above, in the embodiment illustrated in Figure 1, the multiplexer 123 includes one or more of the light source 124, the power source 125, the system controller 126, and the GUI 127. Alternatively, the multiplexer 123 can include more components or fewer components than those specifically illustrated in Figure 1. For example, in certain non-exclusive alternative embodiments, the multiplexer 123 can be designed without the GUI 127. Still alternatively, one or more of the light source 124, the power source 125, the system controller 126, and the GUI 127 can be provided within the catheter system 100 without the specific need for the multiplexer 123.

In some embodiments, the multiplexer 123 can include a two-channel splitter design. The guide bundle 122 can include a manual positioning mechanism that is mounted on an optical breadboard and/or platen. This design enables linear positional adjustment and array tilting by rotating about a channel one light guide 122A axis (not shown in Figure 1). The adjustment method, in other embodiments, can include at least two adjustment steps, 1) aligning the planar positions of the guide beam 124B at Channel 1, and 2) adjusting the light guide bundle 122 to achieve the best alignment at Channel 10.

As illustrated in Figure 1, in certain embodiments, at least a portion of the optical analyzer assembly 142 can be positioned within the multiplexer 123. Alternatively, various components of, or the entire optical analyzer assembly 142, can be positioned outside of, or spaced apart from, the multiplexer 123.

As shown, the multiplexer 123, and the components included therewith, is operatively coupled to the catheter 102, the light guide bundle 122, and the remainder of the catheter system 100. For example, in some embodiments, as illustrated in Figure 1, the multiplexer 123 can include a console connection aperture 148 (also sometimes referred to generally as a "socket") by which the light guide bundle 122 is mechanically coupled to the multiplexer 123. In such embodiments, the light guide bundle 122 can include a guide coupling housing 150 (also sometimes referred to generally as a "ferrule") that houses a portion, e.g., the guide proximal end 122P, of each of the light guides 122A. The guide coupling housing 150 is configured to fit and be selectively retained within the console connection aperture 148 to provide the mechanical coupling between the light guide bundle 122 and the multiplexer 123.

The light guide bundle 122 can also include a guide bundler 152 (or "shell") that brings each of the individual light guides 122A closer together so that the light guides 122A and/or the light guide bundle 122 can be in a more compact form as it extends with the catheter 102 into the blood vessel 108 during use of the catheter system 100. In some embodiments, the light guides 122A leading to the plasma generator 133 can be organized into a light guide bundle 122, including a linear block with an array of precision holes forming a multi-channel ferrule. In other embodiments, the light guide bundle 122 could include a mechanical connector array or block connector that organizes singular ferrules into one of (i) a linear array, (ii) a circular pattern, and (iii) a hexagonal pattern.

The light source 124 can be selectively and/or alternatively coupled in optical communication with each of the light guides 122A, i.e., to the guide proximal end 122P of each of the light guides 122A, in the light guide bundle 122. In particular, the light source 124 is configured to generate light energy in the form of a source beam 124A, such as a pulsed source beam, that can be selectively and/or alternatively directed to and received by each of the light guides 122A in the light guide bundle 122 as an individual guide beam 124B. An optical element 147 can selectively and/or alternatively direct the guide beam 124B to the light guides 122A in the light guide bundle 122 and/or the multiplexer 123. The optical element 147 can include a lens, a focusing lens, a coupling lens, and/or a reflector. Alternatively, the catheter system 100 can include more than one light source 124. For example, in one non-exclusive alternative embodiment, the catheter system 100 can include a separate light source 124 for each light guide 122A in the light guide bundle 122. The light source 124 can be operated at low energies.

The light source 124 can have any suitable design. In certain embodiments, the light source 124 can be configured to provide sub-millisecond pulses of light energy from the light source 124 that are focused onto a small spot in order to couple it into the guide proximal end 122P of the light guide 122A. Such pulses of light energy are then directed and/or guided along the light guides 122A to a location within the balloon interior 146 of the balloon 104, thereby inducing plasma formation (also sometimes referred to herein as a "plasma flash") in the balloon fluid 132 within the balloon interior 146 of the balloon 104, such as via the plasma generator 133 that can be located at the guide distal end 122D of the light guide 122A. In particular, the light emitted at the guide distal end 122D of the light guide 122A energizes the plasma generator 133 to form the plasma within the balloon fluid 132 within the balloon interior 146. The plasma formation causes rapid bubble formation and imparts pressure waves upon the treatment site 106. An exemplary plasma-induced bubble 134 is illustrated in Figure 1.

When the plasma initially forms in the balloon fluid 132 within the balloon interior 146, it emits broad-spectrum electromagnetic radiation. This can be seen as a flash of broad-spectrum light detectable by the naked eye. A portion of the light emitted from the plasma bubble 134 can be transmitted to the distal light receiver 122R at the guide distal end 122D of the light guide 122A and travel back to the guide proximal end 122P where it can be separated, detected, and analyzed through use of the optical analyzer assembly 142. The intensity and timing of the visible light pulse relative to the plasma generating pulse provides an indication that the plasma generator 133 functioned, its energy output, and its functional condition. Visible light flashes may occur in other locations of the light guide 122A if the light guide 122A is damaged or broken. Such other visible light flashes will also be coupled into the light guide 122A and carried back to the guide proximal end 122P. The intensity and timing of these other light pulses provide an indication of damage to or failure of the light guide 122A or the plasma generator 133. In such situations, the optical analyzer assembly 142 can include a safety shutdown system that can be selectively activated to shut down the operation of the catheter system 100.

The configuration of the plasma generator 133 and/or the distal light receiver 122R can, in certain embodiments, further allow ambient light that originates outside of the catheter 102 to be coupled into the guide distal end 122D of the light guide 122A. In one implementation, the optical analyzer assembly 142 monitors for returned ambient light energy that traverses the light guide 122A from the guide distal end 122D to the guide proximal end 122P. If any ambient light energy is present and detected by the optical analyzer assembly 142 in such situations, this is an indication that the catheter 102 is located outside of the body 107 of the patient 109, and the optical analyzer assembly 142 can be configured to lock out the light source 124 accordingly. In particular, in such situations, the safety shutdown system 283 of the optical analyzer assembly 142 can be selectively activated to shut down the operation of the catheter system 100.

In various non-exclusive alternative embodiments, the sub-millisecond pulses of light energy from the light source 124 can be delivered to the treatment site 106 at a frequency of between approximately one hertz (Hz) and 5000 Hz, between approximately 30 Hz and 1000 Hz, between approximately ten Hz and 100 Hz, or between approximately one Hz and 30 Hz. Alternatively, the sub-millisecond pulses of light energy can be delivered to the treatment site 106 at a frequency that can be greater than 5000 Hz or less than one Hz or any other suitable range of frequencies.

It is appreciated that although the light source 124 is typically utilized to provide pulses of light energy, the light source 124 can still be described as providing a single source beam 124A, i.e., a single pulsed source beam.

The light sources 124 suitable for use can include various types of light sources, including lasers, seed sources, and lamps. For example, in certain non-exclusive embodiments, the light source 124 can be an infrared laser that emits light energy in the form of pulses of infrared light. Alternatively, as noted above, the light sources 124, as referred to herein, can include any suitable type of energy source.

Suitable lasers can include short pulse lasers on the sub-millisecond timescale. In some embodiments, the light source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths, and energy levels that can be employed to achieve plasma in the balloon fluid 132 of the catheter 102. In various non-exclusive alternative embodiments, the pulse widths can include those falling within a range, including at least ten ns to 3000 ns, at least 20 ns to 100 ns, or at least one ns to 500 ns. Alternatively, any other suitable pulse width range can be used.

Exemplary nanosecond lasers can include those within the UV to IR spectrum, spanning wavelengths of about ten nanometers (nm) to one millimeter (mm). In some embodiments, the light sources 124 suitable for use in the catheter system 100 can include those capable of producing light at wavelengths of from at least 750 nm to 2000 nm. In other embodiments, the light sources 124 can include those capable of producing light at wavelengths of from at least 700 nm to 3000 nm. In still other embodiments, the light sources 124 can include those capable of producing light at wavelengths of from at least 100 nm to ten micrometers (µm). Nanosecond lasers can include those having repetition rates of up to 200 kHz. In some embodiments, the laser can include a Q-switched thulium:yttrium-aluminum-garnet (Tm:YAG) laser. In other embodiments, the laser can include a neodymium:yttrium-aluminum-garnet (Nd:YAG) laser, holmium:yttrium-aluminum-garnet (Ho:YAG) laser, erbium:yttrium-aluminum-garnet (Er:YAG) laser, excimer laser, helium-neon laser, carbon dioxide laser, as well as doped, pulsed, fiber lasers. In still further embodiments, the light source 124 can include SLEDs that have bandwidths ranging from 13.25 GHz to 18.25 GHz at 1064 nm.

The catheter system 100 can generate pressure waves having maximum pressures in the range of at least one megapascal (MPa) to 100 MPa. The maximum pressure generated by a particular catheter system 100 will depend on the light source 124, the absorbing material, the bubble expansion, the propagation medium, the balloon material, and other factors. In various non-exclusive alternative embodiments, the catheter system 100 can generate pressure waves having maximum pressures in the range of at least approximately two MPa to 50 MPa, at least approximately two MPa to 30 MPa, or at least approximately 15 MPa to 25 MPa.

The pressure waves can be imparted upon the treatment site 106 from a distance within a range from at least approximately 0.1 millimeters (mm) to greater than approximately 25 mm, extending radially from the light guides 122A when the catheter 102 is placed at the treatment site 106. In various non-exclusive alternative embodiments, the pressure waves can be imparted upon the treatment site 106 from a distance within a range from at least approximately ten mm to 20 mm, at least approximately one mm to ten mm, at least approximately 1.5 mm to four mm, or at least approximately 0.1 mm to ten mm extending radially from the light guides 122A when the catheter 102 is placed at the treatment site 106. In other embodiments, the pressure waves can be imparted upon the treatment site 106 from another suitable distance that is different than the foregoing ranges. In some embodiments, the pressure waves can be imparted upon the treatment site 106 within a range of at least approximately two MPa to 30 MPa at a distance from at least approximately 0.1 mm to ten mm. In some embodiments, the pressure waves can be imparted upon the treatment site 106 from a range of at least approximately two MPa to 25 MPa at a distance from at least approximately 0.1 mm to ten mm. Still alternatively, other suitable pressure ranges and distances can be used.

The power source 125 is electrically coupled to and is configured to provide the necessary power to each of the light source 124, the system controller 126, the GUI 127, the handle assembly 128, and the optical analyzer assembly 142. The power source 125 can have any suitable design for such purposes.

The system controller 126 is electrically coupled to and receives power from the power source 125. Additionally, the system controller 126 is coupled to and is configured to control the operation of each of the light source 124, the GUI 127, and the optical analyzer assembly 142. The system controller 126 can include one or more processors or circuits for purposes of controlling the operation of at least the light source 124, the GUI 127, and the optical analyzer assembly 142. For example, the system controller 126 can control the light source 124 for generating pulses of light energy as desired and/or at any desired firing rate. Additionally, the system controller 126 can control and/or operate in conjunction with the optical analyzer assembly 142 to effectively provide continuous real-time monitoring of the performance, reliability, safety, and proper usage of the catheter system 100.

The system controller 126 can further be configured to control the operation of other components of the catheter system 100, such as the positioning of the catheter 102 adjacent to the treatment site 106, the inflation of the balloon 104 with the balloon fluid 132, etc. Further, or in the alternative, the catheter system 100 can include one or more additional controllers that can be positioned in any suitable manner for purposes of controlling the various operations of the catheter system 100. For example, in certain embodiments, an additional controller, and/or a portion of the system controller 126 can be positioned and/or incorporated within the handle assembly 128.

The GUI 127 is accessible by the user or operator of the catheter system 100. Additionally, the GUI 127 is electrically connected to the system controller 126. With such design, the GUI 127 can be used by the user or operator to ensure that the catheter system 100 is effectively utilized to impart pressure onto and induce fractures at the treatment site(s) 106. The GUI 127 can provide the user or operator with information that can be used before, during, and after use of the catheter system 100. In one embodiment, the GUI 127 can provide static visual data and/or information to the user or operator. In addition, or in the alternative, the GUI 127 can provide dynamic visual data and/or information to the user or operator, such as video data or any other data that changes over time during the use of the catheter system 100. In various embodiments, the GUI 127 can include one or more colors, different sizes, varying brightness, etc., which may alert the user or operator. Additionally, or in the alternative, the GUI 127 can provide audio data or information to the user or operator. The specifics of the GUI 127 can vary depending upon the design requirements of the catheter system 100, or the specific needs, specifications, and/or desires of the user or operator.

As shown in Figure 1, the handle assembly 128 can be positioned at or near the proximal portion 114 of the catheter system 100 and/or near the source manifold 136. In this embodiment, the handle assembly 128 is coupled to the balloon 104 and is positioned spaced apart from the balloon 104. Alternatively, the handle assembly 128 can be positioned at another suitable location.

The handle assembly 128 is handled and used by the user or operator to operate, position, and control the catheter 102. The design and specific features of the handle assembly 128 can vary to suit the design requirements of the catheter system 100. In the embodiment illustrated in Figure 1, the handle assembly 128 is separate from, but in electrical and/or fluid communication with one or more of the system controller 126, the light source 124, the fluid pump 138, the GUI 127, and the optical analyzer assembly 142. In some embodiments, the handle assembly 128 can integrate and/or include at least a portion of the system controller 126 within an interior of the handle assembly 128. For example, as shown, in certain such embodiments, the handle assembly 128 can include circuitry (not shown in Figure 1) that can form at least a portion of the system controller 126. In some embodiments, the circuitry can receive electrical signals or data from the optical analyzer assembly 142. Further, or in the alternative, the circuitry can transmit such electrical signals or otherwise provide data to the system controller 126.

In one embodiment, the circuitry can include a printed circuit board (not shown) having one or more integrated circuits, or any other suitable circuitry. In an alternative embodiment, the circuitry can be omitted or can be included within the system controller 126, which in various embodiments can be positioned outside of the handle assembly 128, e.g., within the multiplexer 123. It is understood that the handle assembly 128 can include fewer or additional components than those specifically illustrated and described herein.

As with all embodiments illustrated and described herein, various structures may be omitted from the figures for clarity and ease of understanding. Further, the figures may include certain structures that can be omitted without deviating from the intent and scope of the invention.

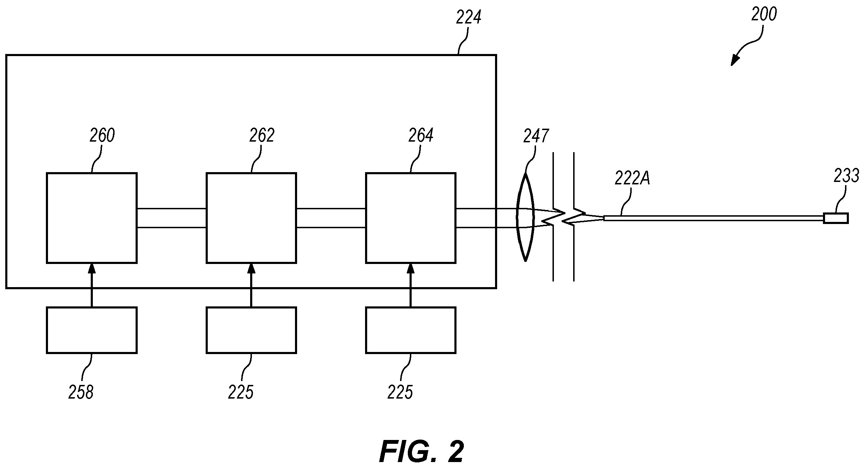

Figure 2 is a simplified schematic diagram of another embodiment of a portion of the catheter system 200. Figure 2 illustrates a non-limiting, non-exclusive example of a master oscillator and power amplifier (MOPA) configuration for the light source 224. In some such embodiments, the MOPA configuration enables a low-power master oscillator to seed the power amplifier with an appropriate pulse to amplify. If the pulse energy is low enough that the amplifier gain is not significantly depleted during the pulse, the temporal shape of the output of the system will closely match the output of the seed laser.

The MOPA configuration also makes it possible to significantly increase the linewidth of the light source 224 while minimizing the nonlinear optical processes and maintaining adequate light energy transmission through the light guide 222A. This configuration provides techniques and methods that significantly increase the linewidth of energy output by the light source 224 by enabling transmission through small diameter light guides 222A with minimal loss.

As illustrated in Figure 2, the catheter system 200 can include the light guide 222A, the light source 224, a power source 225, a plasma generator 233, and an optical element 247. These components can be substantially similar in form, placement, and/or function as previously described with respect to the catheter system 100 illustrated in Figure 1. The catheter system 200 can further include a seed controller 258, a seed source 260, a pre-amplifier 262, and an amplifier 264.

The seed controller 258 controls the operation and/or functionality of the seed source 260. For example, the seed controller 258 can control the output of energy from the seed source 260. The seed controller 258 can control the wavelength, center wavelength, seed pulse shape, frequency, and/or any suitable characteristic of the energy output by the seed source 260. The seed controller 258 can directly modulate the seed source 260. The design and specific features of the seed controller 258 can vary to suit the design requirements of the catheter system 200 and/or the seed source 260.

The seed controller 258 can include one or more processors, microprocessors, and/or circuits for purposes of controlling the operation of at least the seed source 260. The seed controller 258 can include a printed circuit board having one or more integrated circuits, an acousto-optic modulator, or any other suitable circuitry. In an alternative embodiment, the seed controller 258 can be omitted or can be included within the system controller 126 (illustrated in Figure 1), which in various embodiments can be positioned outside of the light source 124, e.g., within the multiplexer 123. It is understood that the seed controller 258 can include fewer or additional components than those specifically illustrated and described herein.

The seed source 260 outputs light energy. The seed source 260 can be in optical communication with the light guide 222A, the optical element 247, the pre-amplifier 262, and the amplifier 264. The seed source 260 can be free space coupled with the pre-amplifier 262, and the amplifier 264 within the light source 224. The seed source 260 can be configured to have a seed offset in center wavelengths that is above and below an amplifier wavelength of the amplifier 264. The seed source 260 can be configured to have a seed pulse shape that is at least partially controlled by (i) directly modulating the seed source 260, or (ii) by an acousto-optic modulator (which can be included within the seed controller 258).

The seed source 260 can have a seed linewidth that is inherent to the seed source. The seed source 260 can be programmable so that the seed linewidth is adjustable to fit the design requirements of the catheter system 200 and/or the seed source 260. In various embodiments, the seed source 260 can be run at a low threshold to length the seed pulse shape. The seed linewidth of the seed source 260 can be adjusted so that a seed center wavelength and a seed overall linewidth is substantially matched to a pre-amplifier center wavelength of the pre-amplifier 262 and/or an amplifier center wavelength of the amplifier 264. By substantially matching the linewidths and/or center wavelengths, the energy conversion of the light energy is improved.

The design and specific features of the seed source 260 can vary to suit the design requirements of the catheter system 200, the light source 224, the light guide 222A, the optical element 247, the pre-amplifier 262, and/or the amplifier 264. The seed source 260 can include a diode, a super-luminescent diode, a diode laser, a programmable semiconductor laser, a gated fiber optic laser, a low power solid-state laser, and/or a modulated distributed feedback laser. It is understood that the seed source 260 can include fewer or additional components than those specifically illustrated and described herein.

The pre-amplifier 262 receives and amplifies the light energy from the seed source 260. In various embodiments, the pre-amplifier 262 can be in optical communication with the light guide 222A, the optical element 247, the seed source 260, and/or the amplifier 264. The pre-amplifier 262 can be powered by the power source 225. The design and specific features of the pre-amplifier 262 can vary to suit the design requirements of the catheter system 200, the light source 224, the light guide 222A, the optical element 247, the seed source 260, and/or the amplifier 264.

The pre-amplifier 262 can include a fiber optic laser, a solid-state laser, a flashlamp, and/or a diode pumped neodymium-doped yttrium aluminum garnet rod. It is understood that the pre-amplifier 262 can include fewer or additional components than those specifically illustrated and described herein. In some embodiments, the pre-amplifier 262 can be entirely omitted from the light source 224 depending on the output energy of the seed source 260 and the design requirements of the catheter system 200, the light source 224, the light guide 222A, the optical element 247, and/or the amplifier 264. In other embodiments, the light source 224 may include a plurality of pre-amplifiers 262.

The amplifier 264 receives and amplifies the light energy from the seed source 260 and/or the pre-amplifier 262. In various embodiments, the amplifier 264 can be in optical communication with the light guide 222A, the optical element 247, the seed source 260, and/or the pre-amplifier 262. The amplifier 264 can be powered by the power source 225. The design and specific features of the amplifier 264 can vary to suit the design requirements of the catheter system 200, the light source 224, the light guide 222A, the optical element 247, the seed source 260, and/or the pre-amplifier 262.

The amplifier 264 can include a high gain stage that is configured to have a high energy output capability, a fiber optic laser, a diode pumped solid-state laser, a gain medium, and/or a flashlamp. The gain medium can include (i) a neodymium-doped yttrium aluminum garnet rod, (ii) a neodymium-doped yttrium aluminum garnet slab, (iii) a neodymium-doped glass, and/or (iv) an erbium-doped yttrium lithium fluoride. The gain medium can be optically coupled to one of a laser diode stack and a flashlamp (e.g., within the amplifier 264). It is understood that the amplifier 264 can include fewer or additional components than those specifically illustrated and described herein. The amplifier 264 can have varying amplifier bandwidths. In some embodiments, the amplifier bandwidth can vary from 1 MHz to 1000 GHz. In other embodiments, the amplifier bandwidth can be less than 1 MHz or greater than 1000 GHz.

The amplifier 264 can include a solid-state high-power amplifier that omits a tuned cavity. The solid-state high-power amplifier can be driven to amplify the light energy to a gain medium linewidth of the gain medium. In some embodiments, when the gain medium includes neodymium-doped yttrium aluminum garnet, the gain medium linewidth is around 0.7 nm. In other embodiments, the light energy solid-state high-power amplifier can be driven to amplify the light energy to a gain medium linewidth of greater than 0.7 nm or less than 0.7 nm.

In some embodiments, the amplifier 264 can be entirely omitted from the light source 224 depending on the output energy of the seed source 260 and the design requirements of the catheter system 200, the light source 224, the light guide 222A, the optical element 247, and/or the pre-amplifier 262. In other embodiments, the light source 224 can include a plurality of amplifiers 264.

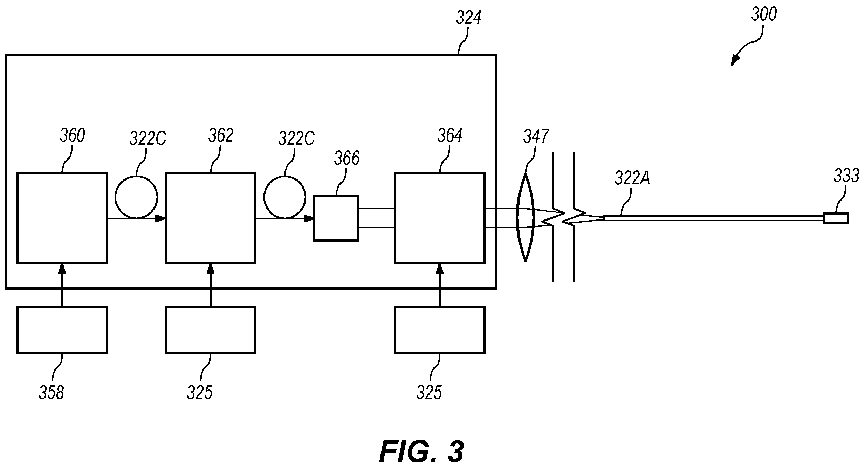

Figure 3 is a simplified schematic diagram of yet another embodiment of a portion of the catheter system 300. As illustrated in Figure 3, the catheter system 300 can include the light guide 322A, the light source 324, a power source 325, a plasma generator 333, and an optical element 347, the seed controller 358, the seed source 360, the pre-amplifier 362, and the amplifier 364. These components can be substantially similar in form, placement, and/or function as previously described with respect to the catheter system 300 illustrated in Figures 1-2. In certain embodiments, the catheter system 300 can further include a plurality of coupling light guides 322C and a collimator 366.

The coupling light guides 322C can optically couple the various components of the light source 324. For example, as shown in Figure 3, a coupling light guide 322C can optically couple the seed source 360 to the pre-amplifier 362, and another coupling light guide 322C can optically couple the pre-amplifier 362 to the collimator 366.

Coupling modes of the various components within the light source 324 can be mixed depending on (i) the design requirements of the catheter system 300 and/or the light source, and (ii) the energy output of the seed source 360, the pre-amplifier 362 and the amplifier 364. For example, free space coupling and coupling light guides 322C can each be utilized within the light source 324 (e.g., as shown in Figure 3).

The design and specific features of the coupling light guide 322C can vary to suit the design requirements of the catheter system 300, the light source 324, the light guide 322A, the optical element 347, the seed source 360, the pre-amplifier 362, the amplifier 364, and/or the collimator 366. The coupling light guide 322C can be a fiber optic cable.

The collimator 366 can collimate the light energy output by the seed source 360, the pre-amplifier 362, and/or the amplifier 364. The collimator 366 can be in optical communication with the light guide 322A, the optical element 347, the seed source 360, the pre-amplifier 362, and/or the amplifier 364. The design and specific features of the collimator 366 can vary to suit the design requirements of the catheter system 300, the light source 324, the light guide 322A, the optical element 347, the seed source 360, the pre-amplifier 362, and/or the amplifier 364. It is understood that the collimator 366 can include fewer or additional components than those specifically illustrated and described herein.

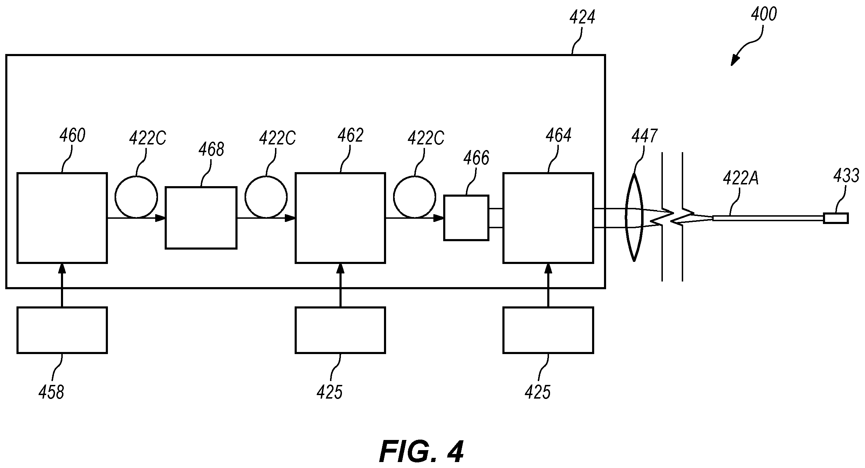

Figure 4 is a simplified schematic diagram of yet another embodiment of a portion of the catheter system 400. As illustrated in Figure 4, the catheter system 400 can include a light guide 422A, a plurality of coupling light guides 422C, a light source 424, a power source 425, a plasma generator 433, and an optical element 447, a seed controller 458, the seed source 460, the pre-amplifier 462, the amplifier 464, and a collimator 466. These components can be substantially similar in form, placement, and/or function as previously described with respect to the catheter system 400 illustrated in Figures 1-3. In certain embodiments, the catheter system 400 can further include a linewidth modifier 468.

The linewidth modifier 468 modifies a linewidth of the light energy output by the seed source 460. In various embodiments, the linewidth modifier 468 can be in optical communication with the light guide 422A, the optical element 447, the seed source 460, the pre-amplifier 462, and/or the amplifier 464. The linewidth modifier 468 and the seed source 460 can work in conjunction to (i) increase a seed linewidth of the seed source 460, (ii) improve amplification of the light energy, and (iii) minimize Stimulated Brillouin Scattering (SBS) in the light guide 422A.

The design and specific features of the linewidth modifier 468 can vary to suit the design requirements of the catheter system 400, the light guide 422A, the light source 424, the optical element 447, the seed source 460, the pre-amplifier 462, and/or the amplifier 464. The linewidth modifier 468 can include a band-limiting filter and/or a fiber-optic Bragg grating, as non-limiting, non-exclusive examples. It is understood that linewidth modifier 468 can include fewer or additional components than those specifically illustrated and described herein.

Lasers

The lasers suitable for use herein can include various types of lasers, including lasers and lamps. Suitable lasers can include short pulse lasers on the submillisecond timescale. In some embodiments, the laser can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths, and energy levels that can be employed to achieve plasma in the balloon fluid of the catheters illustrated and/or described herein. In various embodiments, the pulse widths can include those falling within a range, including from at least 10 ns to 200 ns. In some embodiments, the pulse widths can include those falling within a range including from at least 20 ns to 100 ns. In other embodiments, the pulse widths can include those falling within a range including from at least 1 ns to 5000 ns.

Exemplary nanosecond lasers can include those within the UV to IR spectrum, spanning wavelengths of about 10 nanometers to 1 millimeter. In some embodiments, the lasers suitable for use in the catheter systems herein can include those capable of producing light at wavelengths of from at least 750 nm to 2000 nm. In some embodiments, the lasers can include those capable of producing light at wavelengths of from at least 700 nm to 3000 nm. In some embodiments, the lasers can include those capable of producing light at wavelengths of from at least 100 nm to 10 micrometers ($\mu$m). Nanosecond lasers can include those having repetition rates of up to 200 kHz. In some embodiments, the laser can include a Q-switched thulium:yttrium-aluminum-garnet (Tm:YAG) laser. In some embodiments, the laser can include a neodymium:yttrium-aluminum-garnet (Nd:YAG), holmium:yttrium-aluminum-garnet (Ho:YAG), erbium:yttrium-aluminum-garnet (Er:YAG), excimer laser, helium-neon laser, carbon dioxide laser, as well as doped, pulsed, fiber lasers.

Pressure Waves

The catheters illustrated and/or described herein can generate pressure waves having maximum pressures in the range of at least 1 megapascal (MPa) to 100 MPa. The maximum pressure generated by a particular catheter will depend on the laser, the absorbing material, the bubble expansion, the propagation medium, the balloon material, and other factors. Such factors can include:

(i) Pulse energy can be the primary factor in determining the size of the bubble generated and its ability to fracture calcified lesions. Clinical effectiveness is tied directly to mechanical energy and not the acoustic shockwave that precedes the mechanical bubble. Energy has an inverse cubic relationship with:

$$R \propto \sqrt[3]{E/p} \, .$$

(ii) Pulse width and envelope shape play lesser roles. These factors impact the overall conversion efficiency and the amplitude of the acoustic shockwave that precedes the mechanical bubble.

(iii) Energy delivered - delivering more energy into the region where plasma is generated on the relaxation timescale of the phenomenon improves the conversion efficiency-packing more light energy into the plasma before the bubble begins to form increases conversion efficiency and peak acoustic energy.

(iv)Temporal stretching of the light energy pulse is an effective way to reduce surface irradiance below the damage threshold for light guide material while maintaining total energy in the pulse, thereby maximizing bubble size.

In some embodiments, the catheters illustrated and/or described herein can generate pressure waves having maximum pressures in the range of at least 2 MPa to 50 MPa. In other embodiments, the catheters illustrated and/or described herein can generate pressure waves having maximum pressures in the range of at least 2 MPa to 30 MPa. In yet other embodiments, the catheters illustrated and/or described herein can generate pressure waves having maximum pressures in the range of at least 15 MPa to 25 MPa. In some embodiments, the catheters illustrated and/or described herein can generate pressure waves having peak pressures of greater than or equal to 1 MPa, 2 MPa, 3 MPa, 4 MPa, 5 MPa, 6 MPa, 7 MPa, 8 MPa, 9 MPa, 10 MPa, 11 MPa, 12 MPa, 13 MPa, 14 MPa, 15 MPa, 16 MPa, 17 MPa, 18 MPa, 19 MPa, 20 MPa, 21 MPa, 22 MPa, 23 MPa, 24 MPa, or 25 MPa, 26 MPa, 27 MPa, 28 MPa, 29 MPa, 30 MPa, 31 MPa, 32 MPa, 33 MPa, 34 MPa, 35 MPa, 36 MPa, 37 MPa, 38 MPa, 39 MPa, 40 MPa, 41 MPa, 42 MPa, 43 MPa, 44 MPa, 45 MPa, 46 MPa, 47 MPa, 48 MPa, 49 MPa, or 50 MPa,. It is appreciated that the catheters illustrated and. described herein can generate pressure waves having operating pressures or maximum pressures that can fall within a range, wherein any of the forgoing numbers can serve as the lower or upper bound of the range, provided that the lower bound of the range is a value less than the upper bound of the range.

Therapeutic treatment can act via a fatigue mechanism or a brute force mechanism. For a fatigue mechanism, operating pressures would be about at least 0.5 MPa to 2 MPa, or about 1 MPa. For a brute force mechanism, operating pressures would be about at least 20 MPa to 30 MPa, or about 25 MPa. Pressures between the extreme ends of these two ranges may act upon a treatment site using a combination of a fatigue mechanism and a brute force mechanism.

The pressure waves described herein can be imparted upon the treatment site from a distance within a range from at least 0.01 millimeters (mm) to 25 mm, extending radially from a longitudinal axis of a catheter placed at a treatment site. In some embodiments, the pressure waves can be imparted upon the treatment site from a distance within a range from at least 1 mm to 20 mm, extending radially from a longitudinal axis of a catheter placed at a treatment site. In other embodiments, the pressure waves can be imparted upon the treatment site from a distance within a range from at least 0.1 mm to 10 mm, extending radially from a longitudinal axis of a catheter placed at a treatment site. In yet other embodiments, the pressure waves can be imparted upon the treatment site from a distance within a range from at least 1.5 mm to 4 mm, extending radially from a longitudinal axis of a catheter placed at a treatment site. In some embodiments, the pressure waves can be imparted upon the treatment site from a range of at least 2 MPa to 30 MPa at a distance from 0.1 mm to 10 mm. In some embodiments, the pressure waves can be imparted upon the treatment site from a range of at least 2 MPa to 25 MPa at a distance from 0.1 mm to 10 mm. In some embodiments, the pressure waves can be imparted upon the treatment site from a distance that can be greater than or equal to 0.1 mm, 0.2 mm, 0.3 mm, 0.4 mm, 0.5 mm, 0.6 mm, 0.7 mm, 0.8 mm, or 0.9 mm, 1 mm, 2 mm, 3 mm, 4 mm, 5 mm, 6 mm, 7 mm, 8 mm, 9 mm, or 10 mm, or can be an amount falling within a range between, or outside the range of any of the foregoing.

By shaping the temporal form of the optical pulse to have a fast rise time and minimal overshoot (ideally approaching a square wave), the efficiency for generating the pressure wave can be improved, and the amount of energy that can be delivered in a given time interval can be increased while decreasing the peak laser intensity to remain below the damage threshold of the optical fiber.

The present technology is also directed toward methods for treating a treatment site within or adjacent to a vessel wall, with such methods utilizing the devices disclosed herein.

It should be noted that, as used in this specification and the appended claims, the singular forms "a," "an," and "the" include plural referents unless the content and/or context clearly dictates otherwise. It should also be noted that the term "or" is generally employed in its sense, including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

As used herein, the recitation of numerical ranges by endpoints shall include all numbers subsumed within that range, inclusive (e.g., 2 to 8 includes 2, 2.1, 2.8, 5.3, 7, 8, etc.).

It is recognized that the figures shown and described are not necessarily drawn to scale, and that they are provided for ease of reference and understanding, and for relative positioning of the structures.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope herein.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions, and sub-combinations thereof. It is therefore intended that the following appended claims and claims hereafter introduced are interpreted to include all such modifications, permutations, additions, and sub-combinations as are within their scope, and no limitations are intended to the details of construction or design herein shown.

## Claims

1. A catheter system for treating a treatment site within or adjacent to a vessel wall or a heart valve, the catheter system comprising:
   a light guide (322A, 422A) that is configured to selectively receive light energy a light source (324, 424) configured to generate the light energy, the light source being in optical communication with the light guide; the light source including (i) a seed source (360, 460) configured to output the light energy, (ii) a pre-amplifier (362, 462) configured to receive the light energy from the seed source, the pre-amplifier being in optical communication with the seed source, (iii) an amplifier (364, 464) configured to receive the light energy from the pre-amplifier, the amplifier being in optical communication with the pre-amplifier and the light guide, and (iv) a collimator (366, 466) configured to collimate the light energy output by the pre-amplifier, the collimator being in optical communication with the pre-amplifier and the amplifier.

2. The catheter system of claim 1 further comprising a seed controller configured to control the seed source.

3. The catheter system of any one of claims 1-2 further comprising an optical element that is configured to direct the light energy into the light guide.

4. The catheter system of any one of claims 1-3 wherein the seed source includes one of a diode laser, a programmable semiconductor laser, a gated fiber optic laser, and a low power solid-state laser.

5. The catheter system of any one of claims 1-4 wherein the seed source, the pre-amplifier, and the amplifier are free-space coupled within the light source.

6. The catheter system of any one of claims 1-5 wherein the seed source is optically coupled to the pre-amplifier with a first coupling light guide, and the pre-amplifier is optically coupled to the amplifier with a second coupling light guide.

7. The catheter system of any one of claims 1-6 wherein the pre-amplifier includes one of a fiber optic laser, a solid-state laser, a flashlamp, and a diode pumped neodymium-doped yttrium aluminum garnet rod.

8. The catheter system of any one of claims 1-7 wherein the amplifier includes one of a high gain stage that is configured to have a high energy output capability, a fiber optic laser, a diode pumped solid-state laser, and a flashlamp.

9. The catheter system of any one of claims 1-8 wherein the amplifier includes a gain medium including one of (i) a neodymium-doped yttrium aluminum garnet rod, (ii) a neodymium-doped yttrium aluminum garnet slab, (iii) a neodymium-doped glass, and (iv) an erbium-doped yttrium lithium fluoride, the gain medium being optically coupled to one of a laser diode stack and a flashlamp.

10. The catheter system of any one of claims 1-9 wherein the light source further includes a linewidth modifier configured to modify a linewidth of the light energy output by the seed source.

11. The catheter system of claim 10 wherein the linewidth modifier is one of a band-limiting filter and a fiber-optic Bragg grating.

12. The catheter system of any one of claims 10-11 wherein the seed source and the linewidth modifier work in conjunction to (i) increase a seed linewidth of the seed source, (ii) improve amplification of the light energy, and (iii) minimize Stimulated Brillouin Scattering in the light guide.

13. The catheter system of any one of claims 10-12 wherein the seed source, the linewidth modifier, the pre-amplifier, and the amplifier are positioned inside the light source.

14. The catheter system of any one of claims 10-13 wherein the linewidth modifier is positioned between the seed source and the pre-amplifier inside the light source.

15. The catheter system of any one of claims 10-14 wherein the collimator is positioned between the pre-amplifier and the amplifier inside the light source.

**Patentansprüche**

1. Kathetersystem zur Behandlung einer Behandlungsstelle in oder benachbart zu einer Gefäßwand oder einer Herzklappe, wobei das Kathetersystem aufweist:

    einen Lichtleiter (322A, 422A), der so konfiguriert ist, dass er Lichtenergie selektiv empfängt; eine Lichtquelle (324, 424), die so konfiguriert ist, dass sie die Lichtenergie erzeugt, wobei die Lichtquelle in optischer Kommunikation mit dem Lichtleiter steht; wobei die Lichtquelle aufweist: (i) eine Seed-Quelle (360, 460), die so konfiguriert ist, dass sie die Lichtenergie ausgibt, (ii) einen Vorverstärker (362, 462), der so konfiguriert ist, dass er die Lichtenergie von der Seed-Quelle empfängt, wobei der Vorverstärker in optischer Kommunikation mit der Seed-Quelle steht, (iii) einen Verstärker (364, 464), der so konfiguriert ist, dass er die Lichtenergie vom Vorverstärker empfängt, wobei der Verstärker in optischer Kommunikation mit dem Vorverstärker und dem Lichtleiter steht, und (iv) einen Kollimator (366, 466) der so konfiguriert ist, dass er die vom Vorverstärker ausgegebene Lichtenergie kollimiert, wobei der Kollimator in optischer Kommunikation mit dem Vorverstärker und dem Verstärker steht.

2. Kathetersystem nach Anspruch 1, ferner mit einer Seed-Steuerung, die so konfiguriert ist, dass sie die Seed-Quelle steuert.

3. Kathetersystem nach einem der Ansprüche 1 bis 2, ferner mit einem optischen Element, das so konfiguriert ist, dass es die Lichtenergie in den Lichtleiter richtet.

4. Kathetersystem nach einem der Ansprüche 1 bis 3, wobei die Seed-Quelle einen Diodenlaser, einen programmierbaren Halbleiterlaser, einen gattergesteuerten Faseroptiklaser oder einen leistungsarmen Festkörperlaser aufweist.

5. Kathetersystem nach einem der Ansprüche 1 bis 4, wobei die Seed-Quelle, der Vorverstärker und der Verstärker innerhalb der Lichtquelle freiraumgekoppelt sind.

6. Kathetersystem nach einem der Ansprüche 1 bis 5, wobei die Seed-Quelle durch einen ersten Koppellichtleiter mit dem Vorverstärker optisch gekoppelt ist und der Vorverstärker durch einen zweiten Koppellichtleiter mit dem Verstärker optisch gekoppelt ist.

7. Kathetersystem nach einem der Ansprüche 1 bis 6, wobei der Vorverstärker einen Faseroptiklaser, einen Festkörperlaser, eine Blitzlampe oder einen Dioden-gepumpten Neodym-dotierten Yttrium-Aluminium-Garnet-Stab aufweist.

8. Kathetersystem nach einem der Ansprüche 1 bis 7, wobei der Verstärker eine Hochleistungsstufe, die so konfiguriert ist, dass sie ein energiereiches Ausgabevermögen hat, einen Faseroptiklaser, einen Dioden-gepumpten Festkörperlaser oder eine Blitzlampe aufweist.

9. Kathetersystem nach einem der Ansprüche 1 bis 8, wobei der Verstärker über ein Verstärkungsmedium verfügt, das aufweist: (i) einen Neodym-dotierten Yttrium-Aluminium-Garnet-Stab, (ii) eine Neodym-dotierte Yttrium-Aluminium-Garnet-Platte, (iii) ein Neodym-dotiertes Glas oder (iv) ein Erbium-dotiertes Yttrium-Lithium-Fluorid, wobei das Verstärkungsmedium mit einem Laserdiodenstapel oder einer Blitzlampe optisch gekoppelt ist.

10. Kathetersystem nach einem der Ansprüche 1 bis 9, wobei die Lichtquelle ferner einen Linienbreitenmodifikator aufweist, der so konfiguriert ist, dass er eine Linienbreite der durch die Seed-Quelle ausgegebenen Lichtenergie modifiziert.

11. Kathetersystem nach Anspruch 10, wobei der Linienbreitenmodifikator ein Bandbegrenzungsfilter oder ein faseroptisches Bragg-Gitter ist.

12. Kathetersystem nach einem der Ansprüche 10 bis 11, wobei die Seed-Quelle und der Linienbreitenmodifikator zusammenarbeiten, um (a) eine Seed-Linienbreite der Seed-Quelle zu vergrößern, (ii) die Verstärkung der Lichtenergie zu verbessern und (iii) stimulierte Brillouin-Streuung im Lichtleiter zu minimieren.

**13.** Kathetersystem nach einem der Ansprüche 10 bis 12, wobei die Seed-Quelle, der Linienbreitenmodifikator, der Vorverstärker und der Verstärker innerhalb der Lichtquelle positioniert sind.

**14.** Kathetersystem nach einem der Ansprüche 10 bis 13, wobei der Linienbreitenmodifikator zwischen der Seed-Quelle und dem Vorverstärker innerhalb der Lichtquelle positioniert ist.

**15.** Kathetersystem nach einem der Ansprüche 10 bis 14, wobei der Kollimator zwischen dem Vorverstärker und dem Verstärker innerhalb der Lichtquelle positioniert ist.

**Revendications**

**1.** Système de cathéter pour traiter un site de traitement à l'intérieur de ou adjacent à une paroi de vaisseau ou à une valve cardiaque, le système de cathéter comprenant :

un guide de lumière (322A, 422A) qui est configuré pour recevoir sélectivement une énergie lumineuse ;
une source lumineuse (324, 424) configurée pour générer l'énergie lumineuse, la source lumineuse étant en communication optique avec le guide de lumière ; la source lumineuse comprenant (i) une source de départ (360, 460) configurée pour émettre l'énergie lumineuse, (ii) un pré-amplificateur (362, 462) configuré pour recevoir l'énergie lumineuse provenant de la source de départ, le pré-amplificateur étant en communication optique avec la source de départ, (iii) un amplificateur (364, 464) configuré pour recevoir l'énergie lumineuse provenant du pré-amplificateur, l'amplificateur étant en communication optique avec le pré-amplificateur et le guide de lumière, et (iv) un collimateur (366, 466) configuré pour collimater l'énergie lumineuse émise par le pré-amplificateur, le collimateur étant en communication optique avec le pré-amplificateur et l'amplificateur.

**2.** Système de cathéter selon la revendication 1, comprenant en outre un contrôleur de départ configuré pour contrôler la source de départ.

**3.** Système de cathéter selon l'une quelconque des revendications 1 à 2, comprenant en outre un élément optique qui est configuré pour diriger l'énergie lumineuse dans le guide de lumière.

**4.** Système de cathéter selon l'une quelconque des revendications 1 à 3, dans lequel la source de départ comprend l'un parmi un laser à diode, un laser semi-conducteur programmable, un laser à fibre optique commandé, et un laser transistorisé de faible puissance.

**5.** Système de cathéter selon l'une quelconque des revendications 1 à 4, dans lequel la source de départ, le pré-amplificateur et l'amplificateur sont couplés en espace libre au sein de la source lumineuse.

**6.** Système de cathéter selon l'une quelconque des revendications 1 à 5, dans lequel la source de départ est couplée optiquement au pré-amplificateur au moyen d'un premier guide de lumière de couplage, et le pré-amplificateur est couplé optiquement à l'amplificateur au moyen d'un second guide de lumière de couplage.

**7.** Système de cathéter selon l'une quelconque des revendications 1 à 6, dans lequel le pré-amplificateur comprend l'un parmi un laser à fibre optique, un laser transistorisé, une lampe flash, et un barreau de grenat d'yttrium-aluminium dopé au néodyme pompé par diode.

**8.** Système de cathéter selon l'une quelconque des revendications 1 à 7, dans lequel l'amplificateur comprend l'un parmi un étage à gain élevé qui est configuré pour avoir une capacité de sortie à haute énergie, un laser à fibre optique, un laser transistorisé pompé par diode, et une lampe flash.

**9.** Système de cathéter selon l'une quelconque des revendications 1 à 8, dans lequel l'amplificateur comprend un milieu à gain comprenant l'un parmi (i) un barreau de grenat d'yttrium-aluminium dopé au néodyme, (ii) une plaque en grenat d'yttrium-aluminium dopé au néodyme, (iii) un verre dopé au néodyme, et (iv) un fluorure de lithium-yttrium dopé à l'erbium, le milieu à gain étant couplé optiquement à l'un parmi un empilement de diodes laser et une lampe flash.

**10.** Système de cathéter selon l'une quelconque des revendications 1 à 9, dans lequel la source lumineuse comprend en outre un modificateur de largeur de raie configuré pour modifier une largeur de raie de l'énergie lumineuse émise par la source de départ.

**11.** Système de cathéter selon la revendication 10, dans lequel le modificateur de largeur de raie est l'un parmi un filtre de limitation de bande et un réseau de Bragg sur fibre optique.

**12.** Système de cathéter selon l'une quelconque des revendications 10 à 11, dans lequel la source de départ et le modificateur de largeur de raie fonctionnent conjointement pour (i) augmenter une largeur de raie de départ de la source de départ, (ii) améliorer l'amplification de l'énergie lumineuse, et (iii) minimiser la diffusion Brillouin stimulée dans le guide de lumière.

13. Système de cathéter selon l'une quelconque des revendications 10 à 12, dans lequel la source de départ, le modificateur de largeur de raie, le pré-amplificateur et l'amplificateur sont positionnés à l'intérieur de la source lumineuse.

14. Système de cathéter selon l'une quelconque des revendications 10 à 13, dans lequel le modificateur de largeur de raie est positionné entre la source de départ et le pré-amplificateur à l'intérieur de la source lumineuse.

15. Système de cathéter selon l'une quelconque des revendications 10 à 14, dans lequel le collimateur est positionné entre le pré-amplificateur et l'amplificateur à l'intérieur de la source lumineuse.

*FIG. 1*

FIG. 2

**FIG. 3**

**FIG. 4**

EP 4 423 863 B1

## REFERENCES CITED IN THE DESCRIPTION

**Patent documents cited in the description**

- US 2015250542 A
- US 2014188094 A1